(19) Europäisches Patentamt
European Patent Office
Office européen des brevets

(11) Numéro de publication: **0 362 031 B1**

(12) **FASCICULE DE BREVET EUROPEEN**

(45) Date de publication de fascicule du brevet: **07.07.93**

(51) Int. Cl.[5]: **C07D 215/40**, C07K 5/06, C07K 5/10, A61K 31/47, A61K 37/02

(21) Numéro de dépôt: **89402593.1**

(22) Date de dépôt: **21.09.89**

Le dossier contient des informations techniques présentées postérieurement au dépôt de la demande et ne figurant pas dans le présent fascicule.

(54) **Composés actifs sur les formes tissulaires de la malaria et procédé de préparation.**

(30) Priorité: **23.09.88 FR 8812441**

(43) Date de publication de la demande:
**04.04.90 Bulletin 90/14**

(45) Mention de la délivrance du brevet:
**07.07.93 Bulletin 93/27**

(84) Etats contractants désignés:
**AT BE CH DE ES FR GB GR IT LI LU NL SE**

(56) Documents cités:
**EP-A- 0 037 388**
**EP-A- 0 286 491**

**J. MED. CHEM., vol. 31, 1988, pages 870-874, American Chemical Society; A. PHILIP et al.: "Peptide derivatives of primaquine as potential antimalarial agent"**

**CHEMICAL ABSTRACTS, vol. 99, no. 17, 24 octobre 1983, page 637, résumé no. 140345k, Columbus, Ohio, US; S. HU et al.: "Synthesis of amino acid and dipeptide derivatives of primaquine"**

(73) Titulaire: **LA REGION WALLONNE**
**Square du Bastion, 1A**
**B-1050 Bruxelles(BE)**

(72) Inventeur: **Pirson, Philippe**
**25, avenue de Burbure**
**B-1970 Wezembeek-Oppem(BE)**
Inventeur: **Falmagne, Jean-Bernard**
**19, Montagne d'Aisemont**
**B-1300 Wavre(BE)**
Inventeur: **Trouet, André**
**29, Predikherenberg**
**B-3009 Winksele-Herent(BE)**

(74) Mandataire: **Warcoin, Jacques et al**
**Cabinet Régimbeau 26, avenue Kléber**
**F-75116 Paris (FR)**

**EP 0 362 031 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

J. MED. CHEM., vol. 29, 1986, pages 1765-1769, American Chemical Society; J. HOFSTEENGE et al.: "Carrier-linked primaquine in the chemotherapy of malaria"

CHEMICAL ABSTRACTS, vol. 101, no. 6, 6 août 1984, page 318, résumé no. 43522t, Columbus, Ohio, US; A. TROUET et al.: "Cell targeting of primaquine"

CHEMICAL ABSTRACTS, vol. 96, no. 7, 15 février 1982, page 24, résumé no. 45906y, Columbus, Ohio, US; A. TROUET et al.: "Development of new derivatives of primaquine by association with lysosomotropic carriers"

CHEMICAL ABSTRACTS, vol. 94, no. 17, 27 avril 1981, page 66, résumé no. 132282u, Columbus, Ohio, US; P. PIRSON et al.: "Antimalarial activity of liposomal primaquine and peptidic derivatives of primaquine"

JOURNAL OF PHARMACEUTICAL SCIENCES, vol. 76, no. 2, février 1987, pages 134-140, American Pharmaceutical Association; T. LAAKSO et al.: "Biodegradable microspheres VI: Lysosomal release of covalently bound antiparastitic drugs from starch microparticles"

## Description

La présente invention s'inscrit dans le domaine du traitement thérapeutique de la malaria humaine, notamment des formes tissulaires.

Les 2,5 milliards de personnes vivant dans les zones endémiques où sévit la malaria rappellent l'échec des efforts développés jusqu'à ce jour pour contrôler cette maladie. 400 millions de personnes vivent dans des régions où aucune mesure prophylactique n'est appliquée. L'Organisation Mondiale de la Santé (O.M.S.) estime qu'un million d'enfants de moins de cinq ans meurent chaque année de la maladie, et ce, dans le seul continent africain.

Il y a quatre espèces de plasmodies responsables de la maladie chex l'homme. Ils présentent un cycle évolutif similaire impliquant un vecteur, un moustique femelle du genre Anopheles et un hôte, l'homme. Plasmodium falciparum (fièvre tertiaire maligne) est le plus redoutable ; il est responsable des complications mortelles de la malaria cérébrale et est une des premières causes de la mortalité infantile dans les régions endémiques.

Plasmodium vivax et P. ovale (fièvre tertiaire bénigne) sont moins virulents, mais ils produisent des formes latentes dans les hépatocytes, les hypnozoïtes. Ce sont ces formes qui sont responsables des rechutes classiques pouvant persister durant de longues périodes (deux à cinq ans) après une seule infection. P. malariae est moins répandu ; il est responsable de maladies rénales chroniques telles que les néphrites et les syndromes néphrotiques mortels, et peut persister dans le sang pendant plusieurs années.

Plus précisément, la présente invention concerne la mise au point de nouveaux médicaments actifs sur de la malaria humaine, notamment ses formes tissulaires, à savoir de nouveaux dérivés d'aminoquinoléine, notamment de nouveaux dérivés de la primaquine dont la structure est la suivante :

Formule I

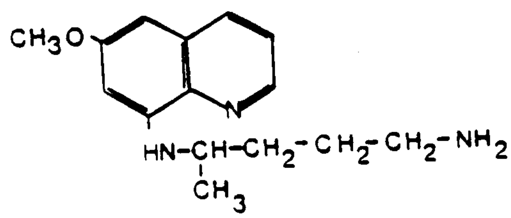

Dans la présente demande de brevet, on entend par aminoquinoléine un dérivé de quinoléine présentant au moins une fonction amine libre, particulièrement les 8-aminoquinoléines parmi lesquelles figurent la primaquine [6-méthoxy-8-(4-amino-1-méthylbutylamino)-quinoléine], mais aussi d'autres 8-aminoquinoléines ayant une fonction amine primaire en fin de chaîne latérale telles que le quinocide [6-méthoxy-8-[4-aminopentylamino)-quinoléine] ou le SN 3883 (6-méthoxy-8-(4-aminobutylamino)-quinoléine].

Mais, il existe également d'autres dérivés de la primaquine, dont les modifications portent sur le noyau quinoléine (en position 2,4,2-4,5,4-5) et sur la chaîne latérale (4-amino-1-alkylbutylamino) qui sont concernés par la présente invention.

Dans les traitements classiques du paludisme les schizontocides tissulaires essentiellement représentés par les 8-aminoquinoléines (primaquine, quinocide) sont responsables de la prophylaxie causale, à savoir la prévention de l'infection sanguine par la destruction des formes tissulaires (exo-érythrocytaires) primaires à P. falciparum et du traitement radical par l'élimination des formes tissulaires secondaires de P. vivax et P. ovale. La primaquine, une 6-méthoxy-8-(4-amino-1-méthylbutylamino)-quinoléine de formule I, introduite il y a plus de trente ans, est cliniquement efficace sur les formes tissulaires persistantes de P. vivax et P. ovale chez l'homme et est toujours un médicament de choix.

Néanmoins, la toxicité de la primaquine limite son emploi à une application clinique contrôlée. L'effet toxique le plus sérieux est l'hémolyse, particulièrement pour les individus présentant une déficience génétique érythrocytaire, par exemple en glucose-6-phosphate déshydrogénase. La méthaemoglobinurie, les troubles gastro-intestinaux ainsi que certains effets sur le système nerveux central sont également des effets toxiques significatifs de la primaquine et autres 8-amino-quinoléines.

Le nombre de médicaments antimalariques possédant une activité thérapeutique prophylactique causale ou curative radicale est extrêmement limité. La primaquine est le seul médicament pour le traitement curatif radical mais les effets toxiques secondaires en limitent sévèrement l'emploi thérapeutique.

Il est à remarquer que seuls les composés appartenant aux familles des 8-aminoquinoléines sont susceptibles de produire une guérison radicale.

Le but de la présente invention est donc de proposer de nouveaux dérivés de quinoléine antimalarique, notamment de nouveaux dérivés de 8-aminoquinoléines possédant un index thérapeutique amélioré.

Autrement dit, le but de la présente invention est de réduire la toxicité intrinsèque desdites quinoléines antimalariques telles que la primaquine et d'augmenter leur activité, notamment pour la primaquine, sur les formes tissulaires hépatiques.

La demande de brevet EP-A-286491 qui fait partie de l'état de la technique au sens de l'article 54(3) de la CBE, décrit des dérivés aminoactifs de la primaquine, notamment la L-glutamyl-Primaquine, qui sont les précurseurs de celle-ci.

L'article Bull. W.H.O., 1981, Vol 59, 449, décrit les dérivés Leu-PQ, Ala-Leu-PQ et Ala-Leu-Ala-Leu-PQ.

L'article de J. Med. Chem., 1988, Vol 31, 870, décrit les dérivés Lys-PQ, Val-Leu-Lys-PQ, D-Val-Leu-Lys-PQ et D-Ala-Leu-Lys-PQ.

On notera que la L-leucine liée à la primaquine a été proposée dans la demande EP-A-37 388 compte tenu de son intérêt en tant que linker ou bras et donc dans le cadre de conjugué vecteur-bras-drogue. En effet, la L-leucyl-primaquine régénère le principe actif libre une fois soumis à la digestion lysosomiale. Toutefois, l'index thérapeutique des dérivés de PQ avec la L-leucine adjacente non conjuguée s'avère moins intéressant que ceux des dérivés qui font l'objet de la présente invention.

La présente invention a en effet pour objet des dérivés d'aminoquinoléine antimalarique, notamment des dérivés de la primaquine, dont la caractéristique essentielle est qu'ils sont représentés schématiquement par la formule PQ-X, dans laquelle :

- PQ représente une 8-aminoquinoléine antimalarique, notamment la primaquine,
- X représente un amino-acide ou un peptide de 2 à 4 amino-acides, l'amino-acide ou le premier amino-acide du peptide adjacent à PQ étant choisi parmi l'acide alpha ou bêta aspartique, l'acide gammaglutamique ou l'acide pyroglutamique.
- la liaison PQ-X étant une liaison covalente peptidique entre le groupement amine libre de PQ et un groupement carboxylique de l'amino-acide ou du premier amino-acide de X.

Dans la présente demande, on entend indiquer par "acide alpha (bêta) aspartique ou gamma glutamique" que l'amino-acide adjacent est lié à PQ sous forme d'ester par sa fonction carboxyle en alpha ou gamma respectivement.

La primaquine agit comme un oxydant et déstabilise la membrane des globules rouges provoquant une hémolyse qui est à l'origine de sa toxicité principale.

L'adjonction d'un amino-acide ou d'un peptide, conformément à la présente invention, réduit la pénétration de la primaquine dans les globules rouges en raison de l'encombrement stérique.

En outre, l'activité de ces nouveaux dérivés est apparue beaucoup plus importante. On a observé également une baisse notable de la toxicité.

Lorsque la liaison covalente entre PQ et X est stable dans le sérum et résistante aux hydrolases lysosomiales, alors, le bénéfice de l'acide aminé ou du peptide joue plus pleinement. Cette condition supplémentaire est en particulier réalisée lorsque X est l'acide bêta aspartique ou gammaglutamique, ou l'acide pyroglutamique.

Ainsi, avantageusement selon l'invention, l'amino-acide ou le premier amino-acide du peptide est l'acide gammaglutamique ou bêta aspartique liés à PQ par leur groupement gamma ou bêta carboxyle, ou encore l'acide pyroglutamique, ou aspartique reliés à PQ par leur groupement alpha carboxyle.

Selon la présente invention, l'amino-acide adjacent à PQ est choisi dans la série D ou L, mais de préférence dans la série L.

Dans un mode de réalisation préférentiel, la présente invention a pour objet de nouveaux dérivés de la primaquine, dont la caractéristique essentielle est qu'ils sont représentés schématiquement par la formule PQ-X, dans laquelle :

- PQ représente une 8-aminoquinoléine antimalarique, notamment la primaquine ;
- X représente l'acide gamma L-glutamique, l'acide L-pyroglutamique, l'acide alpha L-aspartique, ou l'acide bêta L-aspartique ;
- La liaison PQ-X étant une liaison covalente peptidique entre le groupement aminé libre de la primaquine et le groupement carboxylique de X, stable dans le sérum et résistante aux hydrolases lysosomiales.

Il s'agit donc des N-($\alpha$-L-pyroglutamyl)-PQ, N-($\gamma$-L-glutamyl)-PQ, N-($\alpha$-L-aspartyl)-PQ et N-($\beta$-L-aspartyl)-PQ.

Les produits de base PQ ainsi que les produits obtenus PQ-X peuvent se présenter sous forme de leurs sels d'addition avec des acides.

La présente invention a également pour objet un procédé de préparation de ces nouveaux dérivés d'aminoquinoléine antimalarique, notamment des nouveaux dérivés de primaquine.

Dans la caractéristique essentielle du procédé selon la présente invention, on fait réagir par condensation un aminoacide ou un peptide sous forme acide, dont les fonctions amine, et le cas échéant, la fonction carboxyle $\alpha$ ou $\gamma$ ou $\beta$ , sont éventuellement protégées avec l'aminoquinoléine ou un de ses sels portant donc une fonction amine libre, en présence d'un agent de condensation.

Les groupements protecteurs des fonctions amine sont des groupements classiques, mais de préférence on utilise le tertiobutyloxycarbonyle ou le benzyloxycarbonyle.

Les groupements protecteurs de la fonction carboxyle sont des groupements classiques, tels que le terbutyle ou le benzyle.

La fonction acide de l'aminoacide ou du peptide peut aussi être activée par exemple sous forme d'ester avec le N-hydroxysuccinimide.

En général, l'agent de condensation est un carbodiimide comme le dicyclohexylcarbodiimide.

Selon un mode de réalisation particulier du procédé, on part d'un sel d'aminoquinoléine, par exemple du diphosphate de primaquine et de l'acide aminé ou peptide dont la fonction aminée, et le cas échéant la fonction carboxyle $\alpha$ ou $\gamma$ ou $\beta$, sont éventuellement protégées par exemple par un groupe terbutyloxycarbonyle ou benzyloxycarbonyle pour la fonction aminé et le terbutyle ou benzyle pour la fonction carboxyle et on effectue les étapes suivantes :

a) on fait réagir de la N-hydroxysuccinimide ou tout autre groupe activant la fonction acide non protégée de l'acide aminé ou du peptide, sur le dérivé éventuellement protégé de l'acide aminé ou peptide,

b) on fait réagir l'aminoquinoléine, telle que la primaquine, libérée à partir de son sel par exemple par une solution d'ammoniaque, on la fait réagir donc avec par exemple l'ester N-hydroxysuccinimide du dérivé protégé de l'acide aminé ou peptide,

c) le produit brut ainsi obtenu est purifié par chromatographie sur silicagel,

d) les groupements protecteurs le cas échéant sont ensuite clivés en présence d'acide, par exemple l'acide trifluoroacétique pour donner le dérivé PQ-X sous forme de sel par exemple trifluoroacétate, ou sont clivés par hydrogénolyse.

Ce groupement peut en effet être éliminé dans des conditions qui ne détruisent pas la liaison peptidique formée.

Selon une autre caractéristique particulière du procédé, le contre anion trifluoroacétate est échangé en chlorhydrate ou phosphate.

Dans le but d'inhiber les réactions d'oxydation, une autre caractéristique particulière du procédé prévoit que la solution aqueuse contenant le sel de chlorhydrate de dérivés selon la présente invention est traitée par du bisulfite de sodium.

On peut préparer un dérivé PQ-X dans lequel X représente l'acide L pyroglutamique, en transformant un ester en $\gamma$ de l'acide L glutamique, en N-carboxyanhydride, la réaction étant effectuée en présence de phosgène, lequel dérivé N-carboxyanhydride est couplé à PQ par réaction à 0 °C dans le THF pour donner le dérivé (L-pyroglutamyl)-PQ.

Avantageusement, l'ester de l'acide L glutamique est le gamma méthyl glutamate ou le gamma benzyl glutamate.

La présente invention a également pour objet les compositions pharmaceutiques contenant à titre de principe actif les nouveaux dérivés selon la présente invention ou leurs sels pharmaceutiquement acceptables.

D'autres caractéristiques et avantages de l'invention apparaîtront à la lumière des exemples qui vont suivre, faite en référence à la figure 1 qui représente deux voies de synthèse du dérivé N-$\alpha$-L pyroglutamyl-primaquine.

EXEMPLE 1 : Synthèse des dérivés L-pyroglutamyl de la primaquine ou L-$\gamma$-glutamyl

1. Synthése de la N-$\alpha$-L-pyroglutamyl-primaquine (pGLU-PQ)

Le dérivé acide pyroglutamique de la primaquine (PQ) est obtenu selon deux voies, A et B, par condensation de la PQ soit à l'acide pyroglutamique (voie A), soit à un ester activé de l'acide glutamique (voie B). Les deux voies de synthèse sont présentées schématiquement à la figure 1. Ce composé est obtenu sous forme base (P.M.: 370,43) d'un sel monochlorhydrate (P.M.: 406,89) et d'un sel monophosphate (P.M.: 468,44).

1.1. Synthèse selon la voie A:

Le dérivé acide pyroglutamique de la PQ est obtenu en deux étapes à partir de la PQ diphosphate et de l'acide pyroglutamique.

a) Primaquine base

La PQ sous sa forme libre est obtenue à partir de la PQ diphosphate en neutralisant la solution aqueuse contenant le sel de PQ par $NH_4OH$ 25% jusqu'à obtention d'un pH de 8 puis extraction vigoureuse de la PQ base par $CH_2Cl_2$.

b) Activation de l'acide pyroglutamique

L'ester N-hydroxysuccinimide de l'acide L-pyroglutamique est obtenu par réaction de l'acide pyroglutamique, dissous dans le diméthylformamide ou DMF (1 gr dans 5 ml), avec un équivalent de N-hydroxysuccinimide (891 mgr) et un équivalent de dicyclohexylcarbodiimide (1,6 gr). Le mélange est agité à 4°C pendant 16 heures.

c) L-pyroglutamyl-primaquine

La primaquine base est dissoute dans un minimum de $CH_2Cl_2$ et est ajoutée à l'ester activé à 4°C. Le mélange réactionnel est agité à 4°C pendant 16 heures. La dicyclohexyl urée est filtrée et les solvants sont évaporés.
Le brut de réaction est repris alors dans du $CH_2Cl_2$ et est lavé trois fois avec une solution de $NaHCO_3$ 5% puis trois fois par une solution saturée en NaCl. La phase dichlorométhane est séchée sur $Na_2SO_4$, filtrée et evaporée à sec.
Le produit est élué par le mélange dichlorométhane-méthanol/95-5. Les fractions pures sont rassemblées et les solvants sont évaporés. 1,36 gr de L-pyroglutamyl primaquine sont alors isolés (rendement 47,5%).

1.2. Synthèse selon la voie B:

a) Primaquine base

La PQ sous sa forme libre est obtenue à partir de la PQ diphosphate en neutralisant la solution aqueuse contenant le sol de PQ (9,1 gr dans 100 ml) par l'équivalent de NaOH 1 Molaire (20 ml). Le pH de la solution finale est compris entre 9,5 et 10. La PQ base est alors extraite au $CH_2Cl_2$ (3 x 100 ml). Les phases organiques sont rassembées, séchées par entraînement au toluène ou au tétrahydrofurane (THF).

b) La dérivatisation en N-carboxyanhydride (NCA)

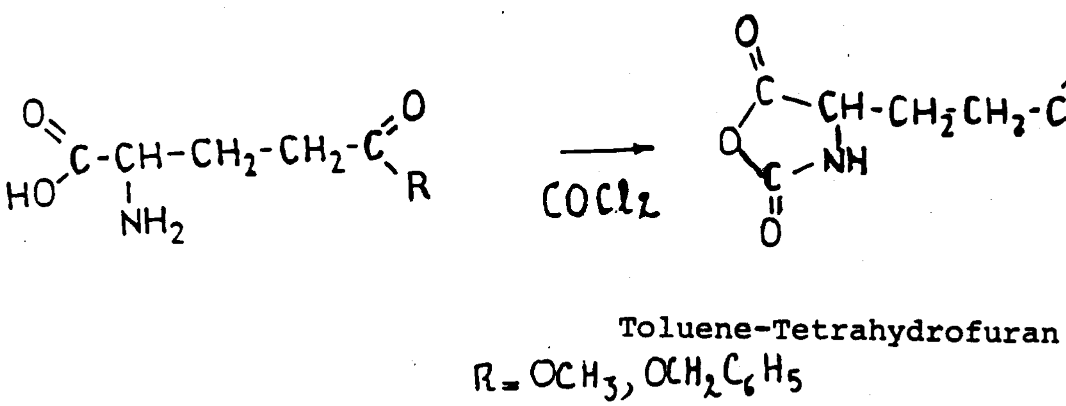


La dérivatisation de l'acide aminé en N-carboxyanhydride est effectuée par la réaction de l'acide aminé avec le phosgène. Plus précisément, 10 gr de gamma méthyl glutamate sont mis en suspension dans 20

ml de THF sec (reflux sur sodium en présence de benzophénone).
D'autre part, une solution de phosgène dans le THF est préparée au départ de phosgène gazeux condensé à basse température et de THF.
La solution de phosgène (40 ml - 2,75 M) est alors ajoutée à la suspension de l'acide aminé dans le THF via un capillaire et avec une surpression en azote.
L'appareil est alors équipé d'un réfrigérant à carboglace, lui-même connecté à deux trappes vides en série, suivi d'un tube plongé dans une solution concentrée de NaOH (capture du phosgène en excès). Le mélange réactionnel est porté à la température de reflux. Cette température est maintenue jusqu'à la dissolution complète de l'acide aminé, soit 2 heures et demi. Les solvants sont évaporés à sec sous vide et à l'abri de l'humidité atmosphérique. Le N-carboxyanhydride est obtenu sous la forme d'une poudre blanche. Le NCA est éventuellement cristallisé par dissolution dans l'acétate d'éthyle (sec) chaud, addition d'un même volume d'éther de pétrole (sec) et refroidissement.

c) Réaction de couplage

Le dérivé NCA, dissous dans le tétrahydrofurane THF (sec), est perfusé lentement (3 heures) dans une solution de primaquine base dans le THF à sec à 0 °C. Du $CO_2$ se dégage du mélange de réaction, indiquant l'ouverture du cycle NCA. Le $CO_2$ est dosé par la formation de $BaCO_3$ lors du passage des dégagements gazeux dans une solution d'hydroxyde de borium. Après réaction, le solvant est évaporé à sec et le brut de la réaction est purifié par chromatographie sur $SiO_2$ ($CH_2Cl_2$ - MeOH).

1.3. Formation des sels:

1° Sel de chlorhydrate
La pglu-PQ est dissoute dans l'eau en présence de deux équivalents d'acide chlorhydrique et lyophilisé. L'opération est répétée une seconde fois.
2° Sel de phosphate
La pglu-PQ est dissoute dans de l'isopropanol et est ajoutée à un équivalent d'acide phosphorique dissous dans de l'isopropanol (1,5% poids/volume). Le sel ne précipite pas dans ces conditions. Le solvant est évaporé et le sel est repris dans l'eau et lyophilisé.

1.4. Propriétés physiques et chimiques:

1.4.1. L-pyroglutamyl-PQ

Pureté par TLC

| | |
|---|---|
| Système: | $CH_2CL_2$MeOH (10/1 en volume) |
| Plaques: | Si-60 |
| Rf: | 0,26 |
| Pureté par TLC: | un seul spot |

Pureté par HPLC

| | |
|---|---|
| Colonne: | silice greffée $C_8$ (Waters). |
| Eluant: | mélange formiate d'ammonium 0,1 M pH 3,85/acétonitrile (70:30 en volume). |
| Détecteur: | U.V. à 363 nm |
| Débit: | 2 ml/min |
| $t_R$: | 4,8 min |
| Pureté par HPLC: | usuellement > 99% en surface; solution 1mgr/ml dans du tampon formiate d'ammonium 0,1 M pH 3,85. |

Point de fusion: N.D.

Pouvoir rotatoire: N.D.

Stabilité: la pGLU-PQ est:

- instable à t° ambiante et à l'air;
- stable à t° ambiante sous athmosphère inerte (argon)

Résonnance magnétique nucléaire:

RMN $^1H$: ($CDCl_3$ - TMS)
RMN $^{13}C$: ($CDCl_3$ - la raie centrale du $CDCl_3$ servant de référence 77.00 ppm).
179.198 (c-5 pglu); 172.233 (c-1 pglu); 159.152 (c-6 PQ); 144.640 (c-8 PQ); 144.106 (c-2 PQ); 135.022 (c-8a PQ); 134.696 (c-4 PQ); 129.704 (c-4a PQ); 121.709 (c-3 PQ); 96.647 (c-7 PQ); 91.507 (c-5 PQ); 56.985 (c-2 pglu); 54.978 (6-$OCH_3$ PQ); 47.589 (c-1′ PQ); 39.248 (c-4′ PQ); 33.539 (c-2′ PQ); 29.147 (c-4 pglu); 25.523 (c-3 pglu); 20.246 (c-5′ PQ).

| Infra-rouge: | |
|---|---|
| $cm^{-1}$ | (%) |
| 3386 | (49%) broad; |
| 1684 | (45%) broad; |
| 1521 | (45%); |
| 1457 | (51%); |
| 1387 | (49%); |
| 1220 | (54%); |
| 1158 | (53%); |
| 822 | (56%); |
| 792 | (56%); |
| 668 | (52%); |

Masse:

FAB +: 371.2 $(M + 1)^+$ 175.1 $(c_{10} H_{10} N_2 O)^+$
FAB -: 369.1$(M - 1)^+$

2. Synthése de la N-γ-L-glutamyl-primaquine (gGLU-PQ)

Le dérivé acide γ-glutamique de la primaquine est obtenu selon la voie de synthèse décrite ci-dessous, sous forme d'un sel dichlorhydrate (P.M. 388,45 + 72,92 = 461,38) et d'un sel monophosphate (P.M.: 468,44).

2.1. Synthèse

Le dérivé acide glutamique de la PQ est obtenu en deux étapes à partir de la PQ diphosphate et de l'acide L-glutamique dont la fonction aminée est protégée soit par un groupe de tertbutyloxycarbonyle (N-α-t-BOC), soit par un groupe benzyloxycarbonyle (N-α-CBZ). La fonction α-carboxyle est protégée soit par un groupe t-butyle, soit par un groupe benzyle.

a) Primaquine base

La primaquine sous sa forme libre est obtenue à partir de la PQ diphosphate en neutralisant la solution aqueuse contenant le sel de PQ par $NH_4OH$ 25% jusqu'à obtention d'un pH de 8 puis extraction vigoureuse de la PQ base par $CH_2Cl_2$.

b) Activation de l'amino-acide protégé

L'ester N-hydroxysuccinimide de l'acide glutamique protégé est obtenu par réaction de 5,9 mM de la N-α-t-BOC-L-GLU-α-t-butyle (1,8 gr) - ou de la N-α-cbz-L-Glu-α-o-benzyle - avec 5,9 mM de N-OH-succinimide (0,68 gr) et 5,9 mM de dicyclohexylcarbodiimide (1,21 gr) dissous à 0 °C dans de l'acétate d'éthyle.

c) N-α-t-BOC-α-t-butyl-γ-L-Glutamyl-PQ

Après 16 heures de réaction entre l'acide glutamique protégé et la NHS à 0 °C, on ajoute 5,9 mmoles (1,53 gr) de PQ base. Après 17 heures, la dicyclohexylurée est filtrée et le solvant chassé sous vide.
L'huile brune obtenue est dissoute dans le $CH_2Cl_2$ et lavée à l'eau. La phase organique est séchée sur sulfate de sodium, filtrée et évaporée. Le produit est purifié sur gel de silice en utilisant comme éluant un mélange dichorométhane-méthanol. Les fractions pures sont rassemblées et l'éluant évaporé conduit à un produit plus ou moins cristalline.
Rendement: 65%.

d) Déprotection

1° Déprotection à l'acide trifluoroacétique et formation du sel d'hydrochlorure.
Le composé obtenu précédemment est dissous dans 45 ml d'un mélange 1:1 de dichlorométhane et d'acide trifluoroacétique. Le mélange est agité 4 heures à température ambiante. Les solvants sont évaporés sous vide et le résidu est repris dans de l'eau et lavé plusieurs fois au mélange acétate d'éthyle-éther (50:50 en volume).
La phase aqueuse est récupérée et lyophilisée. La poudre jaune-orange est reprise dans l'eau et on ajoute deux équivalents d'acide chlorhydrique 0,5 N. La solution ainsi obtenue est alors lyophilisée. Le cycle est répété deux fois. Le sel de chlorhydrate est isolé et gardé sous argon.
2° Déprotection par hydrogénolyse et formation du sel phosphate. La N-α-cbz-α-o-benzyl-L-Glutamyl-Primaquine obtenue précédemment est dissoute dans de l'acétate d'éthyle (100 ml pour 0,01 mole). La solution est complétée par de l'éthanol absolu (150 ml). Le ballon d'un litre est alors purgé à l'argon.
L'hydrogène est introduit après avoir mis le ballon sous vide. Le cycle vide-hydrogène est répété deux fois. La pression en hydrogène est portée à 1,38 Bars (20 Psi).
Après 2 et 4 heures de réaction, le ballon est mis sous vide puis rempli d'hydrogène jusqu'à une pression de 1,38 Bars (20 Psi).
Après 6 ou 7 heures de réaction, l'hydrogène est chassé sous vide et les solvants sont évaporés. La gamma-glutamyl-primaquine est séchée sous vide pendant 16 heures.
Rendement: 100%.

Formation du sel de phosphate

La L-γ-glutamyl-primaquine (0,01 mole) est dissoute dans 60 ml d'éthanol et 10 ml d'acétate d'éthyle. Ce mélange est ajouté à une solution d'acide phosphorique (Merck 573; qualité pour analyse: 85% dans l'isopropanol).
Le volume est porté à 250 ml et le précipité est agité pendant 1 heure. Le précipité est filtré sur membrane Millipore FH sous argon. Le produit est rincé deux fois avec de l'isopropanol, transvasé dans un ballon et séché sous vide +/- 20 heures. 4,4 gr de phosphate de L-γ-glutamyl-primaquine sont ainsi isolés (90%).

2.2. Propriétés physiques et chimiques

2.2.1. L-gammaglutamyl-Primaquine

Pureté par TLC

| | |
|---|---|
| Système: | $CH_2Cl_2$/MeOH (18/2, en volume). |
| Plaques: | Si-60 |
| Rf: | 0,15 |
| Pureté par TLC: | un seul spot |

Pureté par HPLC

| | |
|---|---|
| Colonne: | silice greffée $C_8$ |
| Eluant: | mélange formiate d'ammonium 0,1 M pH 3,85/acétonitrile (70:30 en volume). |
| Détecteur: | U.V. à 363 nm |
| Débit: | 2 ml/min |
| $t_R$: | 2,2 min |
| Pureté par HPLC: | usuellement > 99% |

Point de fusion: N.D.

Pouvoir rotatoire: N.D.

Stabilité: la gGLU-PQ est:

- instable à t° ambiante et à l'air;
- stable à t° ambiante sous atmosphère inerte (argon)

Résonnance magnétique nucléaire:

RMN $^1$H: ($CDCl_3$ - TMS) N.D.
RMN $^{13}$c: ($CDCl_3$ - la raie centrale du $CDCl_3$ servant de référence à 77.00 ppm).

Infra-rouge

Masse

2.2.2. L-gammaglutamyl primaquine.hydrochlorure

Pureté par HPLC:

Usuellement > 99%
Conditions identiques à celles de 4.2.2.1.

2.2.3. L-gammaglutamyl primaquine phosphate

Pureté par HPLC:

Usuellement > 99%
Conditions identiques à celles de 4.2.2.1.

EXEMPLE 2 : ETUDE EXPERIMENTALE IN VITRO

1. Stabilité en présence de sérum

1.1. Protocole

L'influence du sérum sur la stabilité de la primaquine (PQ) et ses dérivés est déterminée en incubant ceux-ci à 37°C à la concentration de 100 µg/ml dans 95% de sérum foetal de veau (SFV, Flow Laboratories), ou du sérum humain et du tampon phosphate salin (PBS 0,15 M, pH 7,2) comme contrôle. Toutes les solutions sont stérilisées par filtration sur filtre Millipore (Millex GV) de 0,20 µm de porosité.

Au cours du temps, des échantillons de 1 ml sont prélevés puis extraits des protéines sériques par précipitation à l'acétonitrile. Pour ce faire, on ajoute à chaque échantillon un volume identique d'acétonitrile. Après mélange vigoureux au Vortex, les échantillons sont laissés 1 heure à 4°C et à l'abri de la lumière puis centrifugés 5 minutes à 12.000 g. Les surnageants sont recueillis puis analysés par HPLC (aliquot de 20 à 50 µl) dans les conditions standard.

1.2. Résultats

Les résultats de la stabilité de la primaquine et de ses deux dérivés en présence de sérum foetal de veau sont repris au tableau 1; les résultats sont exprimés en % de la concentration initiale (temps 0) après extraction.

En présence de sérum, la primaquine est instable; elle se dégrade progressivement pour atteindre 7% après 24 heures. Dans les conditions expérimentales utilisées, on observe un second pic (Y) en HPLC ($t_r$: 4,2 minutes) après 2 heures d'incubation, ne dépassant toutefois pas 10%.

Tableau 1

| Stabilité de la primaquine et de ses dérivés aminoacides en présence de sérum foetal de veau. | | | | | | | |
|---|---|---|---|---|---|---|---|
| COMPOSES [a] | TEMPS D'INCUBATION (heures) [b] | | | | | | |
| | 0 | 1 | 2 | 3 | 6 | 24 | 48 |
| Primaquine PQ | 100(2) | 98(12) | 87(9) | 77(6) | 34(4) | 2 | - |
| Y | 0 | 0 | 5 | 10 | 11 | 5 | - |
| gGLU-PQ PQX | 98(1) | 96(3) | 95(8) | 94(6) | 83(3) | 51(4) | - |
| PQ | 2(1) | 3(0) | 4(2) | 4(2) | 4(1) | 4(3) | - |
| pGLU-PQ PQX | 100(0) | 100(2) | 101(3) | 105(4) | 103(6) | 107(10) | 87 |
| PQ | 0 | 0 | 0 | 0 | 0 | 0 | 0 |

[a]: les composés sont incubés à 37°C en présence de sérum foetal de veau (95% final) puis analysés par spectrophotométrie après HPLC. La pureté initiale des composés, déterminée par HPLC, est respectivement de 99,5%, 98,6% et 99,5%.

[b]: les résultats représentent la concentration relative en primaquine (PQ) et en dérivés (PQ-X) en fonction du temps d'incubation, exprimée en pourcentage de la concentration initiale (temps 0) après extraction (moyenne +/- déviation standard; n = 4, PQ; n = 2, gGLU-PQ et n = 4, pGLU-PQ).

La gGLU-PQ est stable pendant au moins 3 heures, puis une diminution progressive de la concentration relative est observée après 6 heures d'incubation, sans augmentation significative de la concentration en PQ. La pGLU ne subit aucune modification au cours de son incubation dans le sérum. Les deux dérivés ne régénèrent pas de primaquine en présence de sérum après 24 heures ou 48 heures d'incubation.
Des résultats similaires sont obtenus lors d'une incubation des composés en presence de sérum humain. Dans une solution tamponnée (PBS), les composés sont stables, sans aucune modification spectrale significative après 24 heures.

Les résultats obtenus montrent que la liaison covalente entre l'acide aminé et la primaquine est stable et résistante aux enzymes sériques.

2. Hydrolyse en présence d'enzymes lysosomiales

2.1. Protocole:

Les composés, à une concentration finale de 15 µg/ml, sont incubés dans du tampon acétate 50 mM pH 4,5 à 37°C en présence de cystéine 5 mM et d'une fraction d'enzymes lysosomiales (0,15 mg protéine/ml), purifiée à partir de foies de rat.
La fraction lysosomiale purifiée est préparée selon la méthode décrite par Trouet (1974, Méth. Enzymol., 31, 323-334).

Au cours du temps, les échantillons correspondants sont retirés de l'incubation. La réaction enzymatique est arrêtée en ajoutant 10 µl d'HCl 10 M par ml d'échantillon. Un volume de 20 µl est prélevé et analysé directement par HPLC dans les conditions standard.

2.2 Résultats:

L'effet des hydrolases lysosomiales à pH 4,5 sur la PQ et ses dérivés est présenté au tableau 2 ci-dessus.

Tableau 2

| Hydrolyse lysosomiale de la primaquine et de ses dérivés amino-acides. | | | | | | | |
|---|---|---|---|---|---|---|---|
| COMPOSES [a] | TEMPS D'INCUBATION (heures) [b] | | | | | | |
| | 0 | 1 | 2 | 3 | 6 | 24 | 48 |
| Primaquine | 100 | 94 | 96 | 84 | 87 | 68 | - |
| gGLU-PQ PQX | 100 | 104 | 95 | 98 | 101 | 105 | - |
| PQ | 0 | 0 | 0 | 0 | 0 | 0 | - |
| pGLU-PQ PQX | 99 | 94 | 97 | 97 | 96 | 76 | 47 |
| PQ | 1 | 0 | 0 | 0 | 0 | 0 | 0 |

[a]: les composés sont incubés à pH 4,5 en présence d'enzymes lysosomiales (0,15 mg protéine/ml) et de 5 mM cystéine, puis analysés par spectrophotométrie après HPLC. La pureté initiale des composés, déterminée par HPLC, est respectivement de 99,5%, 99,7% et 98,8%.
[b]: les résultats représentent la concentration relative en primaquine (PQ) et dérivés (PQX) en fonction du temps d'incubation, exprimée en pourcentage de la concentration initiale (temps 0) après extraction.

En présence d'enzymes lysosomiales, les deux dérivés de la primaquine ne régénèrent pas la primaquine parentale après 24 ou 48 heures d'incubation.

La liaison covalente entre l'acide aminé et la chaîne latérale de la PQ est résistante à l'hydrolyse lysosomiale. On observe toutefois une diminution progressive de la concentration relative en PQ, atteignant 68% après 24 heures, sans apparition, d'un nouveau pic en HPLC. Le même phénomène est également observé dans le cas de la pGLU-PQ apparaissant seulement après 6 heures d'incubation.

3. Perméabilité des érythrocytes humaines à la primaquine et dérivés

3.1. Protocole:

Du sang humain frais (A+) sur CPDA est lavé deux fois dans du milieu RPMI contenant 20 mM de glucose à pH 7,4, puis resuspendu dans le même milieu à 37°C pour obtenir une suspension à 20% de globules rouges (2 $10^9$ érythrocytes/ml). A 1 ml de cette suspension, on ajoute 1 ml d'une solution de PQ ou de dérivés à 0,250 mM dans le même milieu à 37°C.

Après différents temps d'incubation à 37°C, 0,5 ml d'échantillon est prélevé puis transféré dans un tube Eppendorf contenant 0,4 ml d'huile de silicone (Merck, densité 1,06 g/ml).

Après centrifugation pendant 30 secondes à 12.000 g, le surnageant et l'huile de silicone sont éliminés, tandis que les cellules du culot sont lysées en ajoutant 0,5 ml d'eau. Le composé quinoléinique associé au lysat cellulaire est ensuite extrait par précipitation des protéines cellulaires à l'acétonitrile (0,5 ml), puis analysé par HPLC dans les conditions standard.

La concentration intracellulaire en dérivé quinoléique est obtenue en soustrayant de la concentration totale dans le lysat cellulaire, la concentration en composé présent dans le milieu extracellulaire. Le volume extracellulaire contaminant est évalué à l'aide d'inuline tritiée.

3.2. Résultats:

La PQ est captée rapidement par les érythrocytes (tableau 3). Après 5 minutes d'incubation, 18% de la PQ présente dans le milieu extracellulaire se retrouve dans les globules rouges et cette concentration se maintient pendant au moins une heure.

La gGLU-PQ n'est pas accumulée dans les érythrocytes. La pGLU-PQ s'accumule plus lentement que la PQ; le taux maximal est atteint après 60 minutes et la concentration est similaire à celle de la PQ.

Tableau 3: **Perméabilité des globules rouges humaines à la primaquine et dérivés amino-acides**

| COMPOSES [a] | CONCENTRATION [b] (% concentration initiale) | | |
|---|---|---|---|
| | 5 min. | 10 min. | 60 min. |
| Primaquine | 17,7 (1.2) | 18,0 (3.4) | 18,8 (0,2) |
| gGLU-PQ | 0[c] | 0 | 0 |
| pGLU-PQ | 13,8 | 16,9 | 17,8 |

[a]: les composés sont incubés à une concentration finale de 100 µg/ml en présence de globules rouges humaines à la densité de $10^9$ érythrocytes/ml.

[b]: les résultats représentent la concentration en composés associée aux érythrocytes et exprimée en pourcentage de la concentration initiale (moyenne +/- déviation standard, n = 4 échantillons).

[c]: composé non détectable dans les conditions d'analyse utilisées (seuil de détection = 50 ng).

4. Effet hémolytique et production de méthémoglobuline

Deux des effets toxiques importants de la PQ ont été évalués sur les deux dérivés: l'effet hémolytique et la transformation de l'hémoglobine en méthémoglobine. Le bilan en glutathion (forme réduite et oxydée), intervenant dans le mécanisme de défense de le cellule contre les agents oxydants, a également été étudié.

4.1. Protocole:

Du sang A+ est lavé deux fois dans du tampon phosphate salin 0,15 M à pH 7,4 contenant 20 mM de glucose (GPBS), puis resuspendu à 50% dans la même tampon. A cette suspension, on ajoute un volume égal de composé en solution dans du GPBS à différentes concentrations (test) ou de GPBS (contrôle). L'hématocrite finale de la suspension est de 25%. Après 60 minutes d'incubation à 37 °C, on détermine la concentration en hémoglobine et méthémoglobine sur la suspension totale ainsi que la concentration en hémoglobine présente dans le surnageant après centrifugation (hémoglobine extracellulaire) par les méthodes à la cyanméthémoglobine et à la benzidine (Fairbanks V.F., 1976, in "Fundamentals of Clinical

Chemistry, M.W. Tietz Editors, Saunders"). La teneur en glutathion réduite (GSM) et oxydée (GSSG) est évaluée sur la suspension de globules rouges par la méthode fluorimétrique de Hissin P.J. et Hilf R. (1976, Anal. Biochem., 74, 214-226).

4.2. Résultats

Le tableau 4 représente l'hémolyse, c'est-à-dire la concentration en hémoglobine extracellulaire après 60 minutes d'incubation des érythrocytes en présence de la PQ et de ses dérivés.
La PQ a un effet hémolytique dépendant de la concentration, 4,7% des globules rouges étant lysés après 1 heure en présence de 10 mM de PQ. L'effet hémolytique est également fonction du temps d'incubation (résultat non montré). Les dérivés ont une activité hémolytique comparable à celle de la PQ, représentant à la concentration de 5 mM, une hémolyse de deux fois celle des contrôles.

En ce qui concerne la production de méthémoglobine, celle-ci dépend également de la concentration en dérivé aminoquinoléique. Dans le cas de la PQ, 11,5% d'hémoglobine sont oxydés en méthémoglobine quand les érythrocytes sont en présence de 10 mM de PQ pendant 1 heure (tableau 4).
En présence de la même concentration en dérivé, l'oxydation de l'hémoglobine est similaire à celle de la PQ pour la gGLU-PQ et 1,5 fois moindre pour la pGLU-PQ.

Tableau 4: Effet sur les globules rouges humains de la primaquine et de ses dérivés amino-acides.

| COMPOSES [a] | CONCENTRATION (mM) | HEMOLYSE [b] (%) | METHEMOGLOBINE [b] (%) |
|---|---|---|---|
| Primaquine | 0 (T 0) | 1,3 (0,6) | 1,3 (0,8) |
| | 0 (T 1) | 1,6 (0,4) | 1,4 (0,9) |
| | 1 | 2,1 (1,0) | 1,2 (0,5) |
| | 2 | 2,3 (1,1) | 3,4 (1,8) |
| | 5 | 2,8 (1,2) | 5,8 (0,2) |
| | 10 | 4,7 (1,7) | 11,5 (4,2) |
| gGLU-PQ | 0 (T 0) | 0,20 (0,1) | 1,2 (0,8) |
| | 0 (T 1) | 0,22 (0,05) | 1,0 (0,6) |
| | 1 | 0,27 (0,03) | 1,4 (0,0) |
| | 2 | - | - |
| | 5 | 0,58 (0,05) | 5,3 (0,0) |
| | 10 | - | - |
| pGLU-PQ | 0 (T 0) | 0,22 (0,02) | 1,2 (0,8) |
| | 0 (T 1) | 0,35 (0,03) | 1,0 (0,6) |
| | 1 | 0,32 (0,06) | 0,9 (0,1) |
| | 2 | 0,24 (0,02) | 2,2 (0,9) |
| | 5 | 0,44 (0,02) | 3,9 (0,0) |
| | 10 | 2,34 (0,06) | 6,0 (0,2) |

[a]: les composés, à différentes concentrations, sont incubés 1 heure à 37°C en présence de globules rouges humaines (hématocrite de 25%). Témoin sans composé au temps 0 (T 0) et après 1 heure d'incubation (T 1).

b: les résultats représentent la concentration en hémoglobine extra-cellulaire (hémolyse) et en méthémoglobine (hémoglobine oxydée), exprimée en % de la concentration en hémoglobine totale respective à chaque échantillon (moyenne +/- déviation standard; n = 3 pour la PQ et n = 3 pour la gGLU-PQ et pGLU-PQ).).

EXEMPLE 3 : TOXICITE EXPERIMENTALE CHEZ L'ANIMAL

La toxicité aigüe en fonction de la dose unique administrée est déterminée sur des souris Swiss $OF_1$ femelles de 22 à 25 g. Une série de dilutions de chaque composé est injectée par voie intraveineuse à des groupes de 10 souris (0,1 ml par 10 gr de poids).

La dose léthale pour 50% des souris ($LD_{50}$) est déterminée par régression linéaire des logits du pourcentage des animaux survivants, en fonction du logarithme de la dose injectée (en mg PQ diphosphate équivalent/kg) (Logit Weighted Transformation, Berkson, J., 1953; J. Am. Stat. Assoc. 48, 565-599).

Les résultats repris au tableau 6 montrent que la dose maximale tolérée (MTD), définie comme étant la dose la plus élevée induisant une perte de poids inférieure ou égale à 5% du poids initial pendant une période de 5 jours suivant l'injection i.v., est de 24 mg/kg pour la PQ, 117 mg/kg pour la gGLU-PQ et 126 mg/kg pour la pGLU-PQ.

La dose tuant 50% des animaux ($LD_{50}$) après injection i.v. unique est de 31 mg/kg pour la PQ et respectivement de 169 mg/kg et 159 mg/kg pour les dérivés gGLU-PQ et pGLU-PQ.

Tableau 6

| Toxicité aigüe comparée de la primaquine et de ses dérivés chez la souris après administration i.v. unique. | | |
|---|---|---|
| PRODUIT [a] | MTD [b] (mg/kg) | $LD_{50}$ [c] (mg/kg) |
| Primaquine | 24 (2.1) | 31 (0.7) |
| gGLU-PQ | 117(13) | 169(9) |
| pGLU-PQ | 126(13) | 159(6) |

a: les produits sont administrés par voie i.v. à des groupes de 10 souris Swiss $OF_1$ femelles de 22 à 25 gr, à raison de 5 doses par produit.

b: dose maximale tolérée (MTD), définie comme étant la dose la plus élevée induisant une perte de poids inférieure ou égale à 5% du poids initial pendant une période de 5 jours suivant l'injection i.v.

c: dose léthale pour 50% des souris après administration i.v. unique.

EXEMPLE 4 : ACTIVITE ANTIMALARIQUE CHEZ L'ANIMAL

1. Protocole

La primaquine et ses dérivés sont essayés dans le modèle de la malaria chez le rongeur utilisant l'infection de la souris par Plasmodium berghei, les sporozoïtes provenant de moustiques Anopheles stephensi infectés. Ce modèle, mis au point par Most H., Herman R. & Schoenfeld C. (1967), Am. J. Trop. Med. Hyg. 16, 572-575, permet de déterminer l'activité prophylactique causale (schizontocide tissulaire) de nouveaux composés thérapeutiques.

La procédure utilisée est dérivée de celle de Most H. & Montuori W.A. (1975), Am. J. Trop. Med. Hyg. 24, 179-182. Il s'agit du système P. berghei berghei (ANKA) - Anopheles stephensi - souris Swiss $OF_1$. La souche ANKA de P.b. berghei est entretenue cycliquement sur Anopheles stephensi. La souche de souris Swiss $OF_1$ est particulièrement sensible à P.b. berghei qui produit une infection toujours mortelle.

1.1. Isolement des sporozoïtes des moustiques et infection expérimentale

Les moustiques femelles (80 à 100 individus par préparation) sont débarassés de leurs pattes, ailes et abdomen, puis homogénéisés à 4 °C à l'aide d'un potter en verre, dans du milieu MEM (GIBCO) contenant 10% de sérum foetal de veau. La suspension de sporozoïtes est ensuite purifiée des débris du moustique par deux centrifugations successives (2 min. à 50 g et 5 min. à 200 g) et le surnageant recueilli est conservé à 4 °C. Après comptage dans une chambre de Petroff-Hausser, le nombre de parasites est ajusté par dilution avec le même milieu froid, à la densité de 2 X $10^5$ sporozoïtes/ml.

L'effet résiduel éventuel des médicaments sur les premières formes érythrocytaires (effet schizontocide érythrocytaire) est déterminé par la méthode de Gregory K.G. & Peters W. (Ann. Trop. Med. Parasitol., 1970, 64, 15-24), basée sur le test des 2% de Warhurst.

L'activité prophylactique causale des composés soit la dose permettant de guérir 50% des animaux infectés ($CPD_{50}$) est obtenue par l'analyse des logits de la réponse (exprimée en % de LTS) en fonction de la dose (mg PQ/kg).

L'ensemble de la procédure est toujours effectué dans un maximum de 35 minutes et permet de préserver la viabilité et l'infectivité des sporozoïtes.
Un volume de 0,5 ml de la suspension contenant $10^5$ sporozoïtes est injecté i.v. aux souris Swiss $OF_1$ femelles de 20 à 25 g utilisées pour les tests thérapeutiques.

1.2. Traitements chimiothérapeutiques

Des groupes de 20 à 25 souris infectées comme décrit ci-dessus sont traitées en une seule injection intraveineuse de primaquine diphosphate (PQ) ou de ses deux dérivés sous forme chlorhydrate trois heures après l'inoculation des sporozoïtes. Le volume injecté est ajusté en fonction du poids de la souris, à raison de 0,25 ml pour un poids de 25 gr. Les composés sont dilués dans du tampon phosphate salin (PBS) pH 7,0 et le pH est ajusté à 7,0. Les souris infectées mais non traitées (5 par groupe) reçoivent le tampon seul et servent à contrôler l'infectivité des sporozoïtes.

1.3. Paramètres utilisés pour évaluer l'efficacité de la primaquine et de ses dérivés

Les souris expérimentales sont gardées durant une période de 50 jours. Le développement de la parasitémie est suivi en examinant quotidiennement des frottis de sang, colorés 60 min. au Giemsa à 5% dans du tampon Sörensen à pH 7,2, à partir du 5ème jour et ce, jusqu'au 15ème jour. Le nombre d'animaux infectés peut ainsi être déterminé. Ces résultats permettent d'établir la survie à long terme (LTS, le nombre de souris survivantes non parasitées sur le nombre de souris infectées et traitées).

2. Activité thérapeutique chez l'animal

Les animaux infectés à l'aide de sporozoïtes de P. berghei sont traités trois heures plus tard avec une seule injection des composés par voie i.v. Ce test permet de définir l'activité prophylactique causale des composés, soit la dose permettant de guérir 50% des animaux infectés ($CPD_{50}$). La dose est toujours exprimée en mg de PQ diphosphate par kg.

Les résultats de l'activité prophylactique causale (+ trois heures) de la primaquine et de ses deux dérivés amino-acides sont présentés au tableau 7. Ils indiquent clairement que les deux dérivés sont plus actifs que la PQ, la dose induisant la guérison de 50% des animaux ($CPD_{50}$) étant 2,2 fois moindre pour la gGLU-PQ et 1,9 fois pour la pGLU-PQ que pour la PQ.

La dose minimale effective des deux dérivés est également moins élevée que celle de la PQ. Tandis que la dose totalement curative ne peut être atteinte en une seule injection pour la PQ, en raison de sa toxicité ($DE_{100}$ de la PQ estimée à 54 mg/kg), celles de la gGLU-PQ et de la pGLU-PQ sont atteintes respectivement à 24 mg/kg et 22 mg/kg.

Tableau 7

| Activité prophylactique causale de la primaquine et de ses dérivés amino-acides sur souris $OF_1$ infectées par Plasmodium berghei. | | | | |
|---|---|---|---|---|
| COMPOSE [a] | $CPD_{50}$ [b] (mg/kg) | MED [c] (mg/kg) | $DE_{100}$ c (mg/kg) | INDICE PQ[d] |
| Primaquine | 18,8 (1,3) | 6,5 (2,6) | TOX | 1,00 |
| gGLU-PQ | 8,7 (0,7) | 3,2 (1,1) | 24,2 (9,8) | 2,15 |
| pGLU-PQ | 10,0 (0,7) | 4,6 (1,3) | 21,8 (5,7) | 1,87 |

[a]: les composés sont administrés par voie i.v. trois heures après l'inoculation des sporozoïtes. Les concentrations sont exprimées en mg équivalent PQ diphosphate/kg.
[b]: dose prophylactique causale guérissant 50% des animaux infectés et traités.
[c]: dose minimale effective (MED) et dose totalement curative ($DE_{100}$) calculée par régression linéaire (Weighted Logit Transformation).
[d]: indice PQ (rapport $CPD_{50}$ de la PQ/$CPD_{50}$ du composé.

3. Indice thérapeutique

L'indice thérapeutique est défini comme étant le rapport entre la toxicité, exprimée par la $LD_{50}$, et l'activité thérapeutique, exprimée par la $CPD_{50}$ chez la souris.

L'indice thérapeutique de la PQ est de 1,7 (tableau 8), tandis qu'il est supérieure à 15 et 19 pour la pGLU-PQ et la gGLU-PQ.

Les données expérimentales d'activité dans le modèle de la malaria chez la souris et les données de toxicité indiquent que la gGLU-PQ a un indice thérapeutique au moins 11 fois plus élevé que celui de la PQ, le dérivé étant au moins 5 fois moins toxique et au moins deux fois plus actif que le PQ dans ce modèle expérimental de la malaria.

Il en est de même pour le dérivé pGLU-PQ avec un indice thérapeutique 9 fois supérieur à celui de la PQ, le dérivé étant au moins 5 fois moins toxique et au moins 1,9 fois plus actif que la PQ.

Tableau 8

| Indice thérapeutique de la primaquine et dérivés amino-acides. | | | |
|---|---|---|---|
| PRODUITS [a] | $LD_{50}$ (mg/kg) | $CPD_{50}$ (mg/kg) | TI [b] |
| PQ | 31 (0,7) | 18,8 (1,3) | 1,7 |
| gGLU-PQ | 169 (9) | 8,7 (0,7) | 19,4 |
| pGLU-PQ | 159(6) | 10,0 (0,7) | 15,9 |

[a]: les produits sont inoculés par voie i.v. et les concentrations sont exprimées en équivalents de PQ diphosphate.
[b]: l'indice thérapeutique (TI) est le rapport entre la $LD_{50}$ et la $CPD_{50}$.

EXEMPLE 5 : DERIVE L-ASPARTYL PRIMAQUINE

1. Synthèse

Les dérivés acide aspartique de la primaquine sont obtenus par condensation de la primaquine à un ester activé de l'acide aspartique selon la même voie de synthèse décrite pour les dérivés acide glutamique de la Primaquine.

Le dérivé acide-L-aspartique de la primaquine (ASP-PQ) est synthétisé selon la procédure utilisée pour le dérivé acide gammaglutamique de la primaquine, mais en protégeant la fonction bêta carboxyle au lieu de la fonction alpha carboxyle. Le dérivé acide bêta L aspartique de la primaquine ($\beta$ASP-PQ) est obtenu selon la procédure utilisée pour la gGLU-PQ.

Les composés sont obtenue sous forme base (PM : 374,43) et d'un sel dichlorhydrate (P.M.: 447,35).

2. Toxicité expérimentale chez l'animal

La toxicité aiguë en fonction de la dose unique administrée est déterminée dans les mêmes conditions et selon le protocole de l'exemple 3.

Les résultats repris au tableau 9 montrent que la dose maximale tolérée (MTD), définie comme étant la dose la plus élevée induisant une perte de poids inférieure ou égale à 5% du poids initial pendant une période de 5 jours suivant l'injection i.v., est de 24 mg/kg pour la PQ, 107 mg/kg pour la ASP-PQ et 109 mg/kg pour la βASP-PQ.

La dose tuant 50 % des animaux ($LD_{50}$) après injection i.v. unique est de 31 mg/kg pour la PQ et respectivement de 165 mg/kg et 164 mg/kg pour les dérivés ASP-PQ et βASP-PQ.

Tableau 9

| Toxicité aiguë comparée de la primaquine et de ses dérivés chez la souris après administration i.v. unique. | | |
|---|---|---|
| PRODUIT[a] | MTD [b] (mg/kg) | $LD_{50}$ [c] (mg/kg) |
| Primaquine | 24 (2,1) | 31 (0,7) |
| ASP-PQ | 107,2 (14,2) | 165,5 (5,4) |
| βASP-PQ | 109,1 (13,1) | 164,4 (6,2) |

[a]: les produits sont administrés par voie i.v. à des groupes de 10 souris Swiss $OF_1$ femelles de 22 à 25 g. à raison de 5 doses par produit.
[b]: dose maximale tolérée (MTD), définie comme étant la dose la plus élevée induisant une perte de poids inférieure ou égale à 5 % du poids initial pendant une période de 5 jours suivant l'injection i.v.
[c]: dose léthale pour 50 % des souris après administration i.v. unique.

3. Activité thérapeutique chez l'animal

Les animaux infectés à l'aide de sporozoïtes de P. berghei sont traités trois heures plus tard avec une seule injection des composés par voie i.v. Ce test permet de définir l'activité prophylactique causale des composés, soit la dose permettant de guérir 50 % des animaux infectés ($CPD_{50}$). La dose est toujours exprimée en mg de PQ diphosphate par kg.

Les résultats de l'activité prophylactique causale (+ trois heures) de la primaquine et de ses dérivés amino-acides sont présentés au tableau 10. Ils indiquent clairement que les deux dérivés sont plus actifs que la PQ, la dose induisant la guérison de 50 % des animaux ($CPD_{50}$) étant 1,6 fois moindre pour la ASP-PQ et la βASP-PQ que pour la PQ.

La dose minimale effective des deux dérivés est également moins élevée que celle de la PQ. Tandis que la dose totalement curative ne peut être atteinte en une seule injection pour la PQ, en raison de sa toxicité ($DE_{100}$ de la PA estimée à 54 mg/kg), celles de la ASP-PQ et de la βASP-PQ sont atteintes à 31 mg/kg.

Tableau 10

| Activité prophylactique causale de la primaquine et de ses dérivés amino-acides sur souris $OF_1$ infectées par Plasmodium berghei. | | | | |
|---|---|---|---|---|
| COMPOSE[a] | $CPD_{50}$[b] (mg/kg) | MED[c] (mg/kg) | $DE_{100}$[c] (mg/kg) | INDICE PQ[d] |
| Primaquine | 18,8 (1,3) | 6,5 (2,6) | TOX | 1,00 |
| ASP-PQ | 11,6 (0,9) | 4,4 (1,3) | 31,1 (9,2) | 1,62 |
| βASP-PQ | 11,2 (0,7) | 4,1 (1,1) | 30,6 (5,7) | 1,68 |

[a] : les composés sont administrés par voie i.v. trois heures après l'inoculation des sporozoïtes. Les concentrations sont exprimées en mg équivalent PQ diphosphate/kg.
[b] : dose prophylactique causale guérissant 50 % des animaux infectés et traités.
[c] : dose minimale effective (MED) et dose totalement curative ($DE_{100}$) calculée par régression linéaire (Weighted Logit transformation).
[d] : Indice PQ (rapport $CPD_{50}$ de la PQ/$CPD_{50}$ du composé).

4. Indice thérapeutique

L'indice thérapeutique est défini comme étant le rapport entre la toxicité, exprimée par la $LD_{50}$, et l'activité thérapeutique, exprimée par la $CPD_{50}$ chez la souris.

L'indice thérapeutique de la PQ est de 1,7 (Tableau 11), tandis qu'il est supérieur à 14 pour la ASP-PQ et la βASP-PQ.

Les données expérimentales d'activité dans le modèle de la malaria chez la souris et les données de toxicité indiquent que la ASP-PQ a un indice thérapeutique au moins 8 fois plus élevé que celui de la PQ, le dérivé étant au moins 5 fois moins toxique et 1,6 fois plus actif que la PQ dans ce modèle expérimental de la malaria.

Il en est de même pour le dérivé βASP-PQ avec un indice thérapeutique 8 fois supérieur à celui de la PQ, le dérivé étant au moins 5 fois moins toxique et au moins 1.6 fois plus actif que la PQ.

Tableau 11

| Indice thérapeutique de la primaquine et dérivés amino-acides. | | | |
|---|---|---|---|
| PRODUIT [a] | $LD_{50}$ (mg/kg) | $CPD_{50}$ (mg/kg) | TI [b] |
| Primaquine | 31 (0,7) | 18,8 (1,3) | 1,7 |
| ASP-PQ | 165 (5,4) | 11,6 (0,9) | 14,3 |
| βASP-PQ | 164 (6,2) | 11,2 (0,7) | 14,7 |

[a] : les produits sont inoculés par voie i.v. et les concentrations sont exprimées en équivalents de PQ diphosphate.
[b] : l'indice thérapeutique (TI) est le rapport entre le $LD_{50}$ et la $CPD_{50}$.

**Revendications**

**1.** Dérivés de quinoléines contenant une fonction amine libre et ayant des propriétés antimalariques ou leurs sels d'additions avec des acides, caractérisés en ce qu'ils soient représentés schématiquement par la formule

PQ - X

dans laquelle
- PQ représente une 8-aminoquinoléine antimalarique et

- X est choisi parmi un amino-acide ou un peptide de 2 à 4 amino-acides, l'amino-acide ou le premier amino-acide du peptide étant choisi par l'acide gammaglutamique, l'acide bêta aspartique reliés à un PQ par leur groupement gamma ou beta-carboxyle ou l'acide pyroglutamique et l'acide aspartique reliés à PQ par leur groupement alpha-carboxyle ;
- la liaison PQ-X étant une liaison covalente peptidique entre le groupement amine libre de PQ et un groupement carboxylique de l'acide aminé ou de l'acide aminé terminal de X.

**2.** Dérivés selon la revendication précédente, caractérisés en ce que PQ est la primaquine.

**3.** Procédé de préparation des dérivés selon l'une des revendications précédentes, caractérisé en ce qu'on fait réagir par condensation une fonction carboxyle libre de X, dont les fonctions amine et la fonction alpha, gamma ou bêta carboxyle sont éventuellement protégées, le cas échéant, avec la fonction amine libre de PQ ou un de ses sels, en présence d'un agent de condensation pour former la liaison peptidique PQ-X.

**4.** Procédé selon la revendication 3, caractérisé en ce que le groupement protecteur des fonctions amine est un groupement tertiobutyloxycarbonyle ou benzyloxycarbonyle, et le groupement protecteur des fonctions carboxyles est, le cas échéant, un groupement ter-butyle ou benzyle.

**5.** Procédé selon l'une des revendications 3 ou 4, caractérisé en ce que la fonction acide libre de l'aminoacide ou de peptide est activée sous forme d'ester avec la N-hydroxysuccinimide.

**6.** Procédé selon l'une des revendications 3 à 5, caractérisé en ce qu'on part d'un sel diphosphate de primaquine, et de l'acide aminé (X), dont la fonction aminée et une fonction carboxyle alpha, gamma ou bêta, le cas échéant, sont éventuellement protégées par un groupe terbutyloxycarbonyle ou benzyloxycarbonyle pour la fonction amine, et ter-butyle ou benzyle pour la fonction carboxyle, et on effectue les étapes suivantes :

a) on fait réagir de la N-Hydroxysuccinimide sur le dérivé éventuellement protégé de X,
b) on fait réagir la PQ, libérée à partir de son sel par une solution d'ammoniaque, avec l'ester N-hydroxysuccinimide du dérivé éventuellement protégé de X,
c) le produit brut ainsi obtenu est purifié par chromatographie sur silicagel,
d) les groupements protecteurs, le cas échéant, sont ensuite clivés en présence d'acide, notamment l'acide trifluoroacétique pour donner le dérivé PQ sous forme de sel, notamment trifluoroacétate, ou le composé peut être déprotégée par hydrogénolyse.

**7.** Procédé selon la revendication 6, caractérisé en ce que le contre anion trifluoroacétate est échangé en chlorhydrate, ou phosphate.

**8.** Procédé selon la revendication précédente, caractérisé en ce que le sel chlorhydrate est traité par du bisulfite de sodium.

**9.** Procédé de préparation d'un dérivé PQ-X selon la revendication 1 ou 2, dans lequel X représente l'acide L-pyroglutamique, caractérisé en ce qu on transforme un ester en gamma de l'acide L-glutamique en N-carboxyanhydride$_7$ la réaction étant effectuée en présence de phosgène, lequel dérivé N-carboxyanydride est couplé à PQ par réaction à 0°C dans le THF pour donner le dérivé L-pyroglutamyl-PQ.

**10.** Procédé selon la revendication 9, caractérisé en ce que l'ester de l'acide L glutamique est le gamma méthyl glutamate ou le gamma benzyl glutamate.

**11.** Composition pharmaceutique contenant à titre de principe actif les dérivés selon l'une des revendications 1 ou 2 ou leurs sels pharmaceutiquement acceptables.

## Claims

**1.** New quinoline derivatives containing a free amine group and having antimalarial properties or their addition salts with acids, which derivatives are represented schematically by the formula:

PQ-X

in which

- PQ denotes an antimalarial 8-aminoquinolines, and
- X denotes an amino acid or a peptide of 2 to 4 amino acids, the amino acid or first amino acid of the peptide being chosen from 3 gamma glutamic acid, beta aspartic acid, linked to PQ by their gamma or beta carboxyl group or pyroglutamic acid and aspartic acid linked to PQ by their alpha carboxyl group,
- the PQ-X bond being a covalent peptide bond between the free amine group of PQ and a carboxyl group of the amino acid or the terminal amino acid of X.

**2.** Derivatives according to claim 1 wherein PQ is primaquine.

**3.** A method for preparing the derivatives as claimed in one of the preceding claims, wherein the reaction is performed by condensation of a free carboxyl group of X, in which the amine groups and the $\alpha$- $\beta$- or $\gamma$-carboxyl group are optionally protected, where appropriate, with the free amine group of PQ or one of its salts, in the presence of a condensing agent, to form the PQ-X peptide bond.

**4.** The method as claimed in claim 3, wherein the group protecting the amine groups is a tert-butyloxycarbonyl or benzyloxycarbonyl group, and the group protecting the carboxyl groups is, where appropriate, a tert-butyl or benzyl group.

**5.** The method as claimed in one of claims 3 and 4, wherein the free acid group of the amino acid or peptide is activated in the form of an ester with N-hydroxysuccinimide.

**6.** The method as claimed in one of claims 3 to 5, wherein the starting materials are an aminoquinoline salt (PQ), preferably primaquine diphosphate, and the amino acid or peptide (X), in which the amino group and an $\alpha$-, $\beta$-or $\gamma$-carboxyl group, where appropriate, are optionally protected by a tert-butyloxycarbonyl or benzyloxycarbonyl group for the amine group and a tert-butyl or benzyl group for the carboxyl group, and the following steps are performed:

a) N-hydroxysuccinimide, or any other group activating an unprotected acid group of X, is reacted with the optionally protected derivative of X,

b) PQ, liberated from its salt, for example with ammonia solution, is reacted; it is reacted, for example, with the N-hydroxysuccinimide ester of the optionally protected derivative of X,

c) the crude product thereby obtained is purified by chromatography on silica gel, and

d) the protective groups, where appropriate, are then cleaved off in the presence of acid, in particular trifluoroacetic acid, to give the derivative PQ-X in the form of a salt, in particular trifluoroacetate, or the compound can be deprotected by hydrogenolysis.

**7.** The method as claimed in claim 6, wherein the trifluoroacetate counter-anion is exchanged for hydrochloride or phosphate.

**8.** The method as claimed in the preceding claim, wherein the hydrochloride salt is treated with sodium bisulfite.

**9.** A method for preparing a derivative PQ-X as claimed in claim 1 or 2 in which X denotes L-pyroglutamic acid, wherein an ester at the $\gamma$-position of L-glutamic acid is converted to an N-carboxy anhydride, the reaction being performed in the presence of phosgene, which N-carboxy anhydride derivative is coupled to PQ by reaction at 0 °C in THF to give the derivative (L-pyroglutamyl)-PQ.

**10.** The method as claimed in claim 9, wherein the L-glutamic acid ester is gamma-methyl glutamate or gamma-benzyl glutamate.

**11.** A pharmaceutical composition containing, by way of active principle, the derivatives as claimed in one of claims 1 or 2 or their pharmaceutically acceptable salts.

**Patentansprüche**

1. Chinolinderivate, die eine freie Aminfunktion aufweisen und Antimalaria-Eigenschaften besitzen oder ihre Säureadditionssalze, dadurch gekennzeichnet, daß sie schematisch dargestellt werden durch die Formel

   PQ - X

   worin bedeuten:

   | | |
   |---|---|
   | PQ | ein Antimalaria-8-Aminochinolin und |
   | X | eine Aminosäure oder ein Peptid mit 2 bis 4 Aminosäuren, wobei die Aminosäure oder die erste Aminosäure des Peptids ausgewählt wird aus $\gamma$-Glutaminsäure, $\beta$-Asparaginsäure, die über ihre $\gamma$- oder $\beta$-Carboxylgruppe an ein PQ gebunden sind, oder Pyroglutaminsäure und Asparaginsäure, die über ihre $\alpha$-Carboxyl-Gruppe an PQ gebunden sind; |
   | die Bindung PQ-X | eine kovalente Peptid-Bindung zwischen der freien Amingruppe von PQ und einer Carboxylgruppe der Aminosäure oder der terminalen Aminosäure von X. |

2. Derivate nach dem vorhergehenden Anspruch, dadurch gekennzeichnet, daß es sich bei dem PQ um das Primaquin handelt.

3. Verfahren zur Herstellung der Derivate nach einem der vorhergehenden Ansprüche, dadurch gekennzeichnet, daß man eine freie Carboxylfunktion von X, dessen Aminfunktionen und dessen $\alpha$, $\gamma$- oder $\beta$-Carboxyl-Funktion, falls vorhanden, gegebenenfalls geschützt sind, mit der freien Aminfunktion von PQ oder einem seiner Salze in Gegenwart eines Kondensationsmittels kondensiert zur Bildung der Peptidbindung PQ-X.

4. Verfahren nach Anspruch 3, dadurch gekennzeichnet, daS die Schutzgruppe der Aminfunktionen eine tert-Butyloxycarbonyl- oder Benzyloxycarbonyl-Gruppe ist und daß die Schutzgruppe der Carboxylfunktionen, falls vorhanden, eine tert-Butyl- oder Benzylgruppe ist.

5. Verfahren nach einem der Ansprüche 3 oder 4, dadurch gekennzeichnet, daß die freie Säurefunktion der Aminosäure oder des Peptids in Form eines Esters mit N-Hydroxysuccinimid aktiviert ist.

6. Verfahren nach einem der Ansprüche 3 bis 5, dadurch gekennzeichnet, daß man von einem Diphosphatsalz von Primaquin und der Aminosäure (X) ausgeht, deren Aminfunktion und deren $\alpha$, $\gamma$- oder $\beta$-Carboxylfunktion, falls vorhanden, gegebenenfalls geschützt sind durch eine tert-Butyloxycarbonyl- oder Benzyloxycarbonylgruppe für die Aminfunktion und eine tert-Butyl oder Benzylgruppe für die Carboxylfunktion, und daß man die folgenden Stufen durchführt:
   a) man läßt das N-Hydroxysuccinimid mit dem gegebenenfalls geschützten Derivat von X reagieren,
   b) man läßt das aus seinem Salz durch eine Ammoniak-Lösung freigesetzte PQ mit dem N-Hydroxysuccinimid-Ester des gegebenenfalls geschützten Derivats von X reagieren,
   c) man reinigt das so erhaltene Rohprodukt durch Chromatographie an Silicagel,
   d) man spaltet anschließend in Gegenwart einer Säure, insbesondere der Trifluoressigsäure, die gegebenenfalls vorhandenen Schutzgruppen ab unter Bildung des Derivats PQ in Form seines Salzes, insbesondere des Trifluoroacetats, oder die Verbindung kann durch Hydrogenolyse von den Schutzgruppen befreit werden.

7. Verfahren nach Anspruch 6, dadurch gekennzeichnet, daß das Gegenanion Trifluoroacetat ausgetauscht wird gegen Hydrochlorid oder Phosphat.

8. Verfahren nach dem vorhergehenden Anspruch, dadurch gekennzeichnet, daß das Hydrochloridsalz mit Natriumbisulfit behandelt wird.

9. Verfahren zur Herstellung eines Derivats PQ-X nach Anspruch 1 oder 2, in dem X die L-Pyroglutaminsäure darstellt, dadurch gekennzeichnet, daß man einen $\gamma$-Ester der L-Glutaminsäure in N-Carboxyanhydrid überführt, wobei die Reaktion in Gegenwart von Phosgen durchgeführt wird, daß man das genannte N-Carboxyanhydrid-Derivat durch Umsetzung bei 0 °C in THF an PQ kuppelt unter Bildung

des Derivats L-Pyroglutamyl-PQ.

**10.** Verfahren nach Anspruch 9, dadurch gekennzeichnet, daß es sich bei dem Ester der L-Glutaminsäure um das $\gamma$-Methylglutamat oder das $\gamma$-Benzylglutamat handelt.

**11.** Pharmazeutische Zusammensetzung, die als aktives Prinzip (Wirkstoff) die Derivate nach einem der Ansprüche 1 oder 2 oder ihre pharmazeutisch akzeptablen Salze enthält.

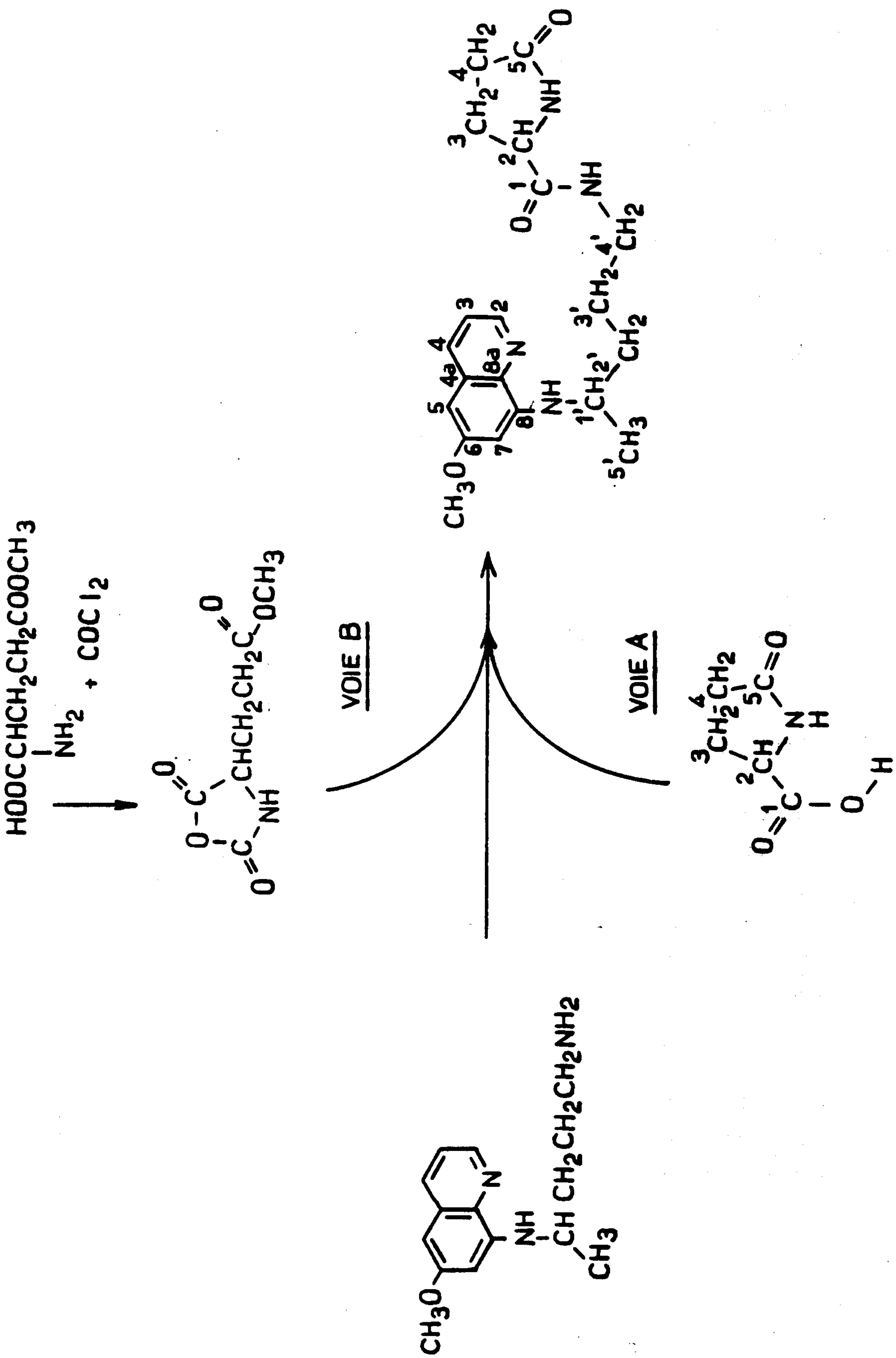
VOIE B
VOIE A